# EUROPEAN PATENT SPECIFICATION

(11) **EP 1 232 183 B1**
(45) Date of publication and mention of the grant of the patent: **18.01.2006**
(21) Application number: 00982219.8
(22) Date of filing: 22.11.2000
(51) Int. Cl.: C07K 14/78, A61K 38/39, C07K 14/705, A61P 35/00

(54) **PEPTIDES HAVING ANTIANGIOGENIC ACTIVITY**
PEPTIDE MIT ANTIANGIOGENER AKTIVITÄT
PEPTIDES PRESENTANT UNE ACTIVITE ANTI-ANGIOGENIQUE

(30) Priority: 22.11.1999 US 447225; 08.11.2000 US 709034
(43) Date of publication of application: 21.08.2002
(73) Proprietor: ABBOTT LABORATORIES, Abbott Park, IL 60064-6050 (US)
(72) Inventor: HAVIV, Fortuna, Deerfield, IL 60015 (US); HENKIN, Jack, Highland Park, IL 60035 (US); BRADLEY, Michael F., Wadsworth, IL 60083 (US); KALVIN, Douglas, M., Buffalo Grove, IL 60089 (US); SCHNEIDER, Andrew, J., Gurnee, IL 60031 (US)
(74) Representative: Modiano, Micaela Nadia
(86) International application number: PCT/US2000/032217
(87) International publication number: WO 2001/038347

(56) References cited:
- EP-A- 0 443 404
- WO-A-98/41542

### SEQUENCE LISTING

<110> Abbott Laboratories
   Haviv, Fortuna
   Henkin, Jack
   Bradley, Michael F.
   Kalvin, Douglas M.
   Schneider, Andrew J.
<120> PEPTIDES HAVING ANTIANGIOGENIC ACTIVITY
<130> 6633.PC.01
<140> US Not Yet Assigned
   <141> 2000-11-08
<150> US 09/447,225
   <151> 1999-11-22
<160> 1
<170> FastSEQ for Windows Version 4.0
<210> 1
   <211> 11
   <212> PRT
   <213> Antiangiogenic Peptide
<220>
   <221> VARIANT
   <222> (1)...(1)
   <223> Xaa = acetyl, 6-methylnicotinyl at position 1
<221> VARIANT
   <222> (2) ... (2)
   <223> Xaa = Sar at position 2
<221> VARIANT
   <222> (3)...(3)
   <223> Xaa = Gly, at position 3
<221> VARIANT
   <222> (4)...(4)
   <223> Xaa = AlloIle, AllylGly, 2-Abu, (IR,4S)AmCyeCO, 5-BrThiAla, 3-ClPheAla, 4-ClPheAla, 3-CNPheAla, Cys(Et), Cys(Me), 2,3-Diapr, 2,4-Diabu, 3,4-diOMePheAla at position 4
<221> VARIANT
   <222> (4) ... (4)
   <223> Xaa = 3-FPheAla, 4-FPheAla, HPheAla, HSer, Lys(Ac), Met(O2), Met(O), 4-MePheAla, 1-Nal, 2-Nal, Orn, PheGly, 3-Pal, 3-ThzAla, 2-ThiAla at position 4
<221> VARIANT
   <222> (4) ... (4)
   <223> Xaa = Ser(Bzl), StyAla, Tic, at position 4
<221> VARIANT
   <222> (5) ... (5)
   <223> Xaa = D-leu, Ile, D-Ile at position 5
<221> VARIANT
   <222> (6) ... (6)
   <223> Xaa = Ser, Thr at position 6
<221> VARIANT
   <222> (7) ... (7)
   <223> Xaa = Gln, Nva, Ser at position 7
<221> VARIANT
   <222> (8)...(8)
   <223> Xaa = Ile at position 8
<221> VARIANT
   <222> (9) ... (9)
   <223> Xaa = Arg at position 9
<221> VARIANT
   <222> (10)...(10)
   <223> Xaa = Pro at position 10
<221> VARIANT
   <222> (11)... (11)
   <223> Xaa = D-Ala-NH₂, NH-ethyl at position 11
<400> 1
   Xaa Xaa Xaa Xaa Xaa Xaa Xaa Xaa Xaa Xaa Xaa
    1               5                   10

## Description

### Technical Field

The invention relates to novel compounds having activity useful for treating conditions which arise or are exacerbated by angiogenesis, pharmaceutical compositions comprising the compounds, methods of treatment using the compounds, and methods of inhibiting angiogensis.

### Background of the Invention

Angiogenesis is the fundamental process by which new blood vessels are formed and is essential to a variety of normal body activities (such as reproduction, development and wound repair). Although the process is not completely understood, it is believed to involve a complex interplay of molecules which both stimulate and inhibit the growth of endothelial cells, the primary cells of the capillary blood vessels. Under normal conditions these molecules appear to maintain the microvasculature in a quiescent state (i.e., one of no capillary growth) for prolonged periods that may last for weeks, or in some cases, decades. However, when necessary, such as during wound repair, these same cells can undergo rapid proliferation and turnover within as little as five days (Folkman, J. and Shing, Y., *The Journal of Biological Chemistry,* **267**(16): 10931-10934, and Folkman, J. and Klagsbrun, M., *Science,* **235**: 442-447 (1987)).

Although angiogenesis is a highly regulated process under normal conditions, many diseases (characterized as "angiogenic diseases") are driven by persistent unregulated angiogenesis. Otherwise stated, unregulated angiogenesis may either cause a particular disease directly or exacerbate an existing pathological condition. For example, ocular neovascularization has been implicated as the most common cause of blindness. In certain existing conditions such as arthritis, newly formed capillary blood vessels invade the joints and destroy cartilage. In diabetes, new capillaries formed in the retina invade the vitreous, bleed, and cause blindness. Growth and metastasis of solid tumors are also angiogenesis-dependent (Folkman, J., *Cancer Research,* **46**: 467-473 (1986), Folkman, J., *Journal of the National Cancer Institute,* **82**: 4-6 (1989)). It has been shown, for example, that tumors which enlarge to greater than 2 mm, must obtain their own blood supply and do so by inducing the growth of new capillary blood vessels. Once these new blood vessels become embedded in the tumor, they provide a means for tumor cells to enter the circulation and metastasize to distant sites, such as the liver, the lung, and the bones (Weidner, N., et. al., *The New England Journal of Medicine*, **324**(1): 1-8 (1991)).

Several angiogenesis inhibitors are currently under development for use in treating angiogenic diseases (Gasparini, G. and Harris, A.L., *J Clin Oncol* **13**(3): 765-782, (1995)). A number of disadvantages have been associated with many of these compounds. A potent angiogenesis inhibitor, for example suramin, can cause severe systemic toxicity in humans at doses required to reach antitumor activity. Other compounds, such as retinoids, interferons, and antiestrogens are safe for human use, but have only a weak anti-angiogenic effect.

Peptide fragments and analogs of thrombospondin have been disclosed in EPA-0 443 404 as having thrombospondin-like activity. A further class of peptides having in the 3-position either natural amino acid residues or their D-isomers have been disclosed in WO 98/41542 as being anti angiogenic drugs.

A novel class of compounds having particularly effective *in vitro* and *in vivo* angiogenesis inhibiting properties, as well as a promising toxicity profile, has been described in commonly-owned U.S. Patent Application Ser. No. 09/316,888, filed May 21, 1999. Copending provisional U.S. Patent Application Ser. No. 60/166,924, filed November 22, 1999, describes N-alkylated peptides having enhanced stability against *in vivo* enzymatic cleavage, improved pharmokinetics, and increased water solubility. Although peptidic compounds inhibiting angiogenesis have been described, it would be desirable to prepare analogs having a favorable toxicity profile which also exhibit improved angiogenesis inhibiting properties.

### Summary of the Invention

The present invention relates to a novel class of peptidic compounds exhibiting antiangiogenic activity. Compounds of the present invention generally have unnatural amino acids in the 3-position of the peptide. The residues in position 3 are structurally novel and can be neutral or charged. The novel substitution of the amino acyl residues in the 3-position of the peptide affords compounds having enhanced properties of angiogenesis inhibition.
- In one aspect, the present invention provides a compound of formula (I) or a pharmaceutically acceptable salt thereof, wherein
   Xaa₁ is selected from the group consisting of
      acetyl, and
      6-methylnicotinyl;
   Xaa₂ is sarcosyl;
   Xaa₃ is glycyl;
   Xaa₄ is selected from the group consisting of
      alloisoleucyl,
      allylglycyl,
      2-aminobutyryl,
      (1R,4S)-1-aminocyclopent-2-ene-4-carbonyl,
      3-(5-bromothien-2-yl)alanyl, 3-(3-chlorophenyl)alanyl,
      3-(4-chlorophenyl)alanyl,
      3-(3-cyanophenyl)alanyl, cysteinyl(S-ethyl),
      cysteinyl(S-methyl),
      2,3-diaminopropionyl;
      2,4-diaminobutanoyl,
      3-(3,4-dimethoxyphenyl)alanyl,
      3-(3-fluorophenyl)alanyl,
      3-(4-fluorophenyl)alanyl,
      homophenylalanyl,
      homoseryl,
      lysyl(N-epsilon-acetyl),
      methionyl(sulfone),
      methionyl(sulfoxide),
      3-(4-methylphenyl)alanyl,
      3-(naphth-1-yl)alanyl,
      3-(naphth-2-yl)alanyl,
      ornithyl,
      phenylglycyl,
      3-(3-pyridyl)alanyl,
      seryl(O-benzyl),
      styrylalanyl,
      1,2,3,4-tetrahydroisoquinoline-3-carbonyl,
      3-(thiazolyl)alanyl,
      3-(thien-2-yl)alanyl, and
      D-3-(thien-2-yl)alanyl ;
   Xaa₅ is selected from the group consisting of
      isoleucyl,
      D-isoleucyl, and
      D-leucyl;
   Xaa₆ is selected from the group consisting of
      seryl, and
      threonyl;
   Xaa₇ is selected from the group consisting of
      glutaminyl,
      norvalyl, and
      seryl;
   Xaa₈ is isoleucyl;
   Xaa₉ is arginyl;
   Xaa₁₀ is prolyl; and
   Xaa₁₁ is selected from the group consisting of
      D-alanylamide, and
      NH-ethyl.

In another aspect, the present invention provides a composition for treating a patient in need of anti-angiogenesis therapy comprising a compound of formula (I) in combination with a pharmaceutically acceptable carrier.

Yet another aspect of the present invention provides the use of a compound of formula (I) for manufacturing a medicament for treating a patient in need of anti-angiogenesis therapy by administration to the patient a therapeutically effective amount of a compound of formula (I).

Still yet another aspect of the present invention provides a composition for the treatment of a disease selected from cancer, arthritis, psoriasis, angiogenesis of the eye associated with infection or surgical intervention, macular degeneration, and diabetic retinopathy comprising a compound of formula (I) in combination with a pharmaceutically acceptable carrier.

In yet another aspect, the present invention provides a method of isolating a receptor from an endothelial cell comprising binding a compound of formula (I) to the receptor to form a peptide receptor complex, isolating the peptide receptor complex, and purifying the receptor.

### Detailed Description of the Invention

### Definition of Terms

As used herein, the singular forms "a", "an", and "the" include plural reference unless the context clearly dictates otherwise.

If used in the present specification the following terms have the meanings indicated:

The term "N-acetylamino," if used herein, refers to NHC(O)CH₃.

The term "acyl," if used herein, refers to an alkyl group attached to the parent molecular moiety through a carbonyl group.

The term "alkoxy," if used herein, refers to an alkyl group attached to the parent molecular moiety through oxygen atom.

The term "alkyl," if used herein, refers to a monovalent group derived from a straight or branched chain saturated hydrocarbon by the removal of a hydrogen atom. Preferred alkyl groups for the invention are C₁-C₆ alkyl groups having from one to six carbon atoms. Alkyl groups of one to three carbon atoms (C₁-C₃ alkyl) are more preferred for the invention.

The term "amino," as used herein, refers to -NH₂.

The term "aryl," if used herein, refers to a mono- or bicyclic carbocyclic ring system having one or two aromatic rings and is exemplified by phenyl, naphthyl, 1,2-dihydronaphthyl, 1,2,3,4-tetrahydronaphthyl, fluorenyl, indanyl, indenyl, and the like.

The term "carbonyl," as used herein, refers to -C(O)-.

The term "carboxy," if used herein, refers to -CO₂H.

The term "cycloalkenyl," if used herein, refers to a non-aromatic cyclic or bicyclic ring system having three to ten carbon atoms and one to three rings, wherein each five-membered ring has one double bond, each six-membered ring has one or two double bonds, each seven- and eight-membered ring has one to three double bonds, and each nine-to ten-membered ring has one to four double bonds. Examples of cycloalkenyl groups include cyclohexenyl, octahydronaphthalenyl, norbornylenyl.

The term "cycloalkyl," if used herein, refers to a saturated monocyclic, bicyclic, or tricyclic hydrocarbon ring system having three to twelve carbon atoms. Examples of cycloalkyl groups include cyclopropyl, cyclopentyl, bicyclo[3.1.1]heptyl, adamantyl.

The term "halo," if used rein, refers to F, Cl, Br, or I.

The term "heterocycle," if used herein, refers to a five-, six-, or seven-membered ring containing one, two, or three heteroatoms independently selected from the group consisting of nitrogen, oxygen, and sulfur. The five-membered ring has zero to two double bonds and the six- and seven-membered rings have zero to three double bonds. The term "heterocycle" also includes bicyclic groups in which the heterocycle ring is fused to an aryl group. The heterocycle groups of the present invention can be attached through a carbon atom or a nitrogen atom in the group. Examples of heterocycles include, but are not limited to, furyl, thienyl, pyrrolyl, pyrrolidinyl, oxazolyl, thiazolyl, imidazolyl, imidazolinyl, pyrazolyl, isoxazolyl, isothiazolyl, piperidinyl, morpholinyl, thiomorpholinyl, piperazinyl, pyridinyl, indolyl, indolinyl, benzothienyl.

The term "hydroxy," as used herein, refers to -OH.

The term "nicotinyl," as used herein, refers to the acyl group derived from nicotinic acid, i.e. pyridine-3-carboxylic acid.

The term "nitrogen protecting group" or "N-protecting group," as used herein, refers to an easily removable group which is known in the art to protect an amino group against undesirable reaction during synthetic procedures and to be selectively removable. The use of nitrogen protecting groups is well known in the art for protecting groups against undesirable reactions during a synthetic procedure and many such protecting groups are known (see, for example, T.H. Greene and P.G.M. Wuts, Protective Groups in Organic Synthesis, 2nd edition, John Wiley & Sons, New York (1991). Examples of N-protecting groups include, are not limited to, acyl groups including acetyl, trifluoroacetyl, acylisothiocyanate, arninocaproyl, benzoyl, and acyloxy groups, including *t-*butoxycarbonyl (Boc) and carbobenzyloxy (Cbz), 9-fluorenylmethoxycarbonyl (Fmoc).

The term "pharmaceutically acceptable ester," if used herein, refers to esters which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than six carbon atoms. Examples of particular esters include formates, acetates, propionates, butyrates, acrylates and ethylsuccinates.

The term "pharmaceutically acceptable prodrugs," if used herein, refers to those prodrugs of the compounds of the present invention which are, within the scope of sound medical judgment, suitable for use in contact with the tissues of humans and lower animals with undue toxicity, irritation, allergic response, commensurate with a reasonable benefit/risk ratio, and effective for their intended use, as well as the zwitterionic forms, where possible, of the compounds of the invention. The term "prodrug" refers to compounds that are rapidly transformed *in vivo* to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

The term "pharmaceutically acceptable salt," as used herein, represents salts or zwitterionic forms of the compounds of the present invention which are water or oil-soluble or dispersible, which are suitable for treatment of diseases without undue toxicity, irritation, and allergic response; which are commensurate with a reasonable benefit/risk ratio, and which are effective for their intended use. The salts can be prepared during the final isolation and purification of the compounds or separately by reacting an amino group with a suitable acid. Representative acid addition salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxyethansulfonate (isethionate), lactate, maleate, mesitylenesulfonate, methanesulfonate, naphthylenesulfonate, nicotinate, 2-naphthalenesulfonate, oxalate, pamoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Also, amino groups in the compounds of the present invention can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting a carboxy group with a suitable base such as the hydroxide, carbonate, or bicarbonate of a metal cation or with ammonia or an organic primary, secondary, or tertiary amine. The cations of pharmaceutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, 1-ephenamine, and N,N'-dibenzylethylenediamine. Other representative organic amines useful for the formation of base addition salts include ethylenediamine, ethanolamine, diethanolamine, piperidine, and piperazine.

The term "pharmaceutically acceptable solvate," if used herein, refers to an aggregate that comprises one or more molecules of the solute, such as a compound of formula (I), with one or more molecules of solvent.

The term "receptor," as used herein, refers to chemical groups or molecules on the cell surface or in the cell interior that have an affinity for a specific chemical group or molecule. Isolation of receptors relevant to the antiangiogenic activity of the peptide of the invention can provide useful diagnostic tools.

The term "shikimyl," as used herein, refers to the acyl residue derived from shikimic acid or [3R-(3α,4α,5β)-3,4,5-trihydroxy]-1-cyclohexene-1-carboxylic acid. A "dihydroshikimyl" group denotes the fully saturated analog of shikimic acid.

The term "succinyl," as used herein, refers to the acyl residue derived from succinic acid or (1,4-dioxobutyl)-1-carboxylic acid.

Unless indicated otherwise by a "D-" prefix, *e.g*. D-Ala or N-Me-D-Ile, the stereochemistry of the α-carbon of the amino acids and aminoacyl residues in peptides described in this specification and the appended claims is the natural or "*L*" configuration. The Cahn-Ingold-Prelog "R" and "S" designations are used to specify the stereochemistry of chiral centers in certain acyl substituents at the N-terminus of the peptides of this invention. The designation "R,S" is meant to indicate a racemic mixture of the two enantiomeric forms. This nomenclature follows that described in R.S. Cahn, *et al., Angew. Chem. Int. Ed Engl.,* **5**,385-415 (1966).

All peptide sequences are written according to the generally accepted convention whereby the α-N-terminal amino acid residue is on the left and the α-C-terminal is on the right. As used herein, the term "α-N-terminal" refers to the free α-amino group of an amino acid in a peptide, and the term "α-C-terminal" refers to the free α-carboxylic acid terminus of an amino acid in a peptide.

For the most part, the names on naturally occurring and non-naturally occurring aminoacyl residues used herein follow the naming conventions suggested by the IUPAC Commission on the Nomenclature of Organic Chemistry and the IUPAC-IUB Commission on Biochemical Nomenclature as set out in "Nomenclature of α-Amino Acids (Recommendations, 1974) " Biochemistry, 14(2), (1975). To the extent that the names and abbreviations of amino acids and aminoacyl residues employed in this specification and appended claims differ from those suggestions, they will be made clear to the reader. Some abbreviations useful in describing the invention are defined below in the following Table 1.

**Table 1**

| Abbreviation | Definition |
|---|---|
| N-Ac-Sar | N-acetylsarcosyl |
| Ala | alanyl |
| β-Ala | β-alanyl |
| AlaNH₂ | alanylamide |
| AlaNH-ethyl | alanyl ethylamide |
| alloIle | alloisoleucyl |
| alloThr | allothreonyl |
| AllylGly | allylglycyl |
| 2-Abu | 2-aminobutyryl |
| (1R,4S)-AmCyeCO | (1R,4S)-1-aminocyclopent-2-ene-4- |
| Aib | 2-aminoisobutyryl |
| AiPheAla | 3-(4-amino-N-isopropylphenyl)alanyl |
| AimPheAla | [(4-amino(N- |
| 4-AmPheAla | 3-(4-aminophenyl)alanyl |
| Arg | arginyl |
| Arg(diethyl) | arginyl(N^{G}N^{G'} diethyl) |
| Asn | asparaginyl |
| Asp | aspartyl |
| AzaGlyNH₂ | azaglycylamide |
| BiPheAla | 3-(4,4'-biphenyl)alanyl |
| 5-BrThiAla | 3-(5-bromothien2-yl)alanyl |
| Gly(*t*-Bu) | *t*-butylglycyl |
| CamdPheAla | 3-(4-carboxyamidophenyl)alanyl |
| 3-ClPheAla | 3-(3-chlorophenyl)alanyl |
| 4-ClPheAla | 3-(4-chlorophenyl)alanyl |
| Cit | citrullyl |
| 3-CNPheAla | 3-(3-cyanophenyl)alanyl |
| Cha | cyclohexylalanyl |
| Cha(Isp) | 3-(cyclohexyl)alanyl(N-isopropyl) |
| Chg | cyclohexylglycyl |
| Cys | cysteinyl |
| Cys(*t*-Bu) | cysteinyl(S-*t*-butyl) |
| Cys(Et) | cysteinyl(S-ethyl) |
| Cys(Me) | cysteinyl(S-methyl) |
| deLeu | dehydroleucyl |
| 2,4-Diabu | 2,4-diaminobutyryl |
| 2,3-Diapr | 2,3-diaminopropionyl |
| 3,4-diFPheAla | 3-(3,4-difluorophenyl)alanyl |
| 3,4-diOMePheAla | 3-(3,4-dimethoxyphenyl)alanyl |
| Gly(Et) | N-ethylglycyl |
| Fmoc | 9-fluorenylmethyloxycarbonyl |
| -3-FPheAla | 3-(3-fluorophenyl)alanyl |
| 4-FPheAla | 3-(4-fluorophenyl)alanyl |
| Gln | glutaminyl |
| Gln(Trt) | glutaminyl(trityl) |
| Glu | glutamyl |
| Gly | glycyl |
| GlyNH₂ | glycylamide |
| GlyNH-ethyl | glycyl ethylamide |
| Gly(pipad) | glycyl[4-piperidinyl(N-amidino)] |
| GuaAla | 3-(guanidino)alanyl |
| 4-GuaPheAla | 3-(4-guanidinophenyl)alanyl |
| His | histidyl |
| His(Trt) | histidyl(trityl) |
| HAla | homoalanyl |
| HArg | homoarginyl |
| HIle | homoisoleucyl |
| HLeu | homoleucyl |
| HPheAla | homophenylalanyl |
| HPro | homoprolyl |
| HSer | homoseryl |
| HSer(*t*-Bu) | homoseryl(O-*t*-butyl) |
| 4-OHMePheAla | 3-(4-hydroxymethylphenyl)alanyl |
| OHPro | hydroxyprolyl |
| Ile | isoleucyl |
| Leu | leucyl |
| Lys | lysyl |
| Lys(Ac) | lysyl(N-epsilon-acetyl) |
| Lys(Isp) | lysyl(N-epsilon-isopropyl) |
| Lys(Nic) | lysyl(N-epsilon-nicotinyl) |
| Met | methionyl |
| Met(O₂) | methionyl(sulfone) |
| Met(O) | methionyl (sulfoxide) |
| N-MeAla | N-methylalanyl |
| 4-MePheAla | 3-(4-methylphenyl)alanyl |
| N-MePro | N-methylprolyl |
| 1-Nal | 3-(naphth-1-yl)alanyl |
| 2-Nal | 3-(naphth-2-yl)alanyl |
| nPenGly | neopentylglycyl |
| 4-NO₂PheAla | 3-(4-nitrophenyl)alanyl |
| NArg | norarginyl |
| Nle | norleucyl |
| Nva | norvalyl |
| OctylGly | octylglycyl |
| Om | ornithyl |
| Orn(Imd) | ornithyl[N-delta-(2-imidazolinyl)] |
| Orn(Isp) | ornithyl(N-delta-isopropyl) |
| Orn(Nic) | ornithyl(N-delta-nicotinyl) |
| Pen | penicillaminyl |
| Pen(Sacme) | penicillaminyl(S-acetamidomethyl) |
| Pen(SBzl) | penicillaminyl(S-benzyl) |
| Pen(SMe) | penicillaminyl(S-methyl) |
| Arg(Pmc) | (N^{G}-2,2,5,7,8-pentamethylchroman-6- |
| PheAla | phenylalanyl |
| PheGly | phenylglycyl |
| Pro | prolyl |
| ProNH-ethyl | prolyl ethylamide |
| PropGly | propargylglycyl |
| 3-Pal | 3-(3-pyridyl)alanyl |
| Glu(pyro) | pyro-glutamyl |
| (pyramid)Ala | [3-pyrrolidinyl(2-N-amidino)]alanyl |
| (pipamid)Ala | [4-piperidinyl(N-amidino)]alanyl |
| Sar | sarcosyl |
| SarNH₂ | sarcosylamide |
| Ser | seryl |
| SerNH₂ | serylamide |
| Ser(Bzl) | seryl(O-benzyl) |
| StyAla | styrylalanyl |
| Tic | 1,2,3,4-tetrahydroisoquinoline-3-carbonyl |
| 3-ThzAla | 3-(3-thiazolyl)alanyl |
| 2-ThiAla | 3-(thien-2-yl)alanyl |
| The | threonyl |
| Thr(Bzl) | threonyl(O-benzyl) |
| Thr(*t*-Bu) | threonyl(O-*t*-butyl) |
| 3-CF₃PheAla | 3-(3-trifluoromethylphenyl)alanyl |
| 3,4,5-TriFPheAla | 3-(3,4,5-trifluorophenyl)alanyl |
| Trp | tryptyl |
| Trp(Boc) | tryptyl(N-*t*-butoxycarbonyl) |
| Tyr | tyrosyl |
| Tyr(*t*-Bu) | tyrosyl(O-*t*-butyl) |
| Tyr(Bzl) | tyrosyl(O-benzyl) |
| Tyr(Et) | tyrosyl(O-ethyl) |
| Val | valyl |

When not found in the table above, nomenclature and abbreviations may be further clarified by reference to the Calbiochem-Novabiochem Corp. *1999 Catalog and Peptide Synthesis Handbook* or the Chem-Impex International, Inc. *Tools for Peptide & Solid Phase Synthesis 1998-1999 Catalogue.*

### Compositions

The compounds of the invention, including but not limited to those specified in the examples, possess anti-angiogenic activity. As angiogenesis inhibitors, such compounds are-useful in the treatment of both primary and metastatic solid tumors, including carcinomas of breast, colon, rectum, lung, oropharynx, hypopharynx, esophagus, stomach, pancreas, liver, gallbladder and bile ducts, small intestine, urinary tract (including kidney, bladder and urothelium), female genital tract, (including cervix, uterus, and ovaries as well as choriocarcinoma and gestational trophoblastic disease), male genital tract (including prostate, seminal vesicles, testes and and germ cell tumors), endocrine glands (including the thyroid, adrenal, and pituitary glands), and skin, as well as hemangiomas, melanomas, sarcomas (including those arising from bone and soft tissues as well as Kaposi's sarcoma) and tumors of the brain, nerves, eyes, and meninges (including astrocytomas, gliomas, glioblastomas, retinoblastomas, neuromas, neuroblastomas, Schwannomas, and meningiomas). Such compounds may also be useful in treating solid tumors arising from hematopoietic malignancies such as leukemias (i.e. chloromas, plasmacytomas and the plaques and tumors of mycosis fungosides and cutaneous T-cell lymphoma/leukemia) as well as in the treatment of lymphomas (both Hodgkin's and non-Hodgkin's lymphomas). In addition, these compounds may be useful in the prevention of metastases from the tumors described above either when used alone or in combination with radiotherapy and/or other chemotherapeutic agents.

Further uses include the treatment and prophylaxis of autoimmune diseases such as rheumatoid, immune and degenerative arthritis; various ocular diseases such as diabetic retinopathy, retinopathy of prematurity, corneal graft rejection, retrolental fibroplasia, neovascular glaucoma, rubeosis, retinal neovascularization due to macular degeneration, hypoxia, angiogenesis in the eye associated with infection or surgical intervention, and other abnormal neovascularization conditions of the eye; skin diseases such as psoriasis; blood vessel diseases such as hemagiomas, and capillary proliferation within atherosclerotic plaques; Osler-Webber Syndrome; myocardial angiogenesis; plaque neovascularization; telangiectasia; hemophiliac joints; angiofibroma; and wound granulation. Other uses include the treatment of diseases characterized by excessive or abnormal stimulation of endothelial cells, including but not limited to intestinal adhesions, Crohn's disease, atherosclerosis, scleroderma, and hypertrophic scars, i.e. keloids. Another use is as a birth control agent, by inhibiting ovulation and establishment of the placenta. The compounds of the invention are also useful in the treatment of diseases that have angiogenesis as a pathologic consequence such as cat scratch disease *(Rochele minalia quintosa)* and ulcers *(Helicobacter pylori).* The compounds of the invention are also useful to reduce bleeding by administration prior to sugery, especially for the treatment of resectable tumors.

The compounds of the invention may be used in combination with other compositions and procedures for the treatment of diseases. For example, a tumor may be treated conventionally with surgery, radiation or chemotherapy combined with a peptide of the present invention and then a peptide of the present invention may be subsequently administered to the patient to extend the dormancy of micrometastases and to stabilize and inhibit the growth of any residual primary tumor. Additionally, the compounds of the invention may be combined with pharmaceutically acceptable excipients, and optionally sustained-release matrices, such as biodegradable polymers, to form therapeutic compositions.

A sustained-release matrix, as used herein, is a matrix made of materials, usually polymers, which are degradable by enzymatic or acid-base hydrolysis or by dissolution. Once inserted into the body, the matrix is acted upon by enzymes and body fluids. A sustained-release matrix desirably is chosen from biocompatible materials such as liposomes, polylactides (polylactic acid), polyglycolide (polymer of glycolic acid), polylactide co-glycolide (copolymers of lactic acid and glycolic acid) polyanhydrides, poly(ortho)esters, polypeptides, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide (co-polymers of lactic acid and glycolic acid).

When used in the above or other treatments, a therapeutically effective amount of one of the compounds of the present invention may be employed in pure form or, where such forms exist, in pharmaceutically acceptable salt form. By a "therapeutically effective amount" of the compound of the invention is meant a sufficient amount of the compound to treat an angiogenic disease, (for example, to limit tumor growth or to slow or block tumor metastasis) at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific compound employed; and like factors well known in the medical arts. For example, it is well within the skill of the art to start doses of the compound at levels lower than those required to achieve the desired therapeutic effect and to gradually increase the dosage until the desired effect is achieved.

Alternatively, a compound of the present invention may be administered as pharmaceutical compositions containing the compound of interest in combination with one or more pharmaceutically acceptable excipients. A pharmaceutically acceptable carrier or excipient refers to a non-toxic solid, semi-solid or liquid filler, diluent, encapsulating material or formulation auxiliary of any type. The compositions may be administered parenterally, intracisternally, intravaginally, intraperitoneally, topically (as by powders, ointments, drops or transdermal patch), rectally, or bucally. The term "parenteral" as used herein refers to modes of administration which include intravenous, intramuscular, intraperitoneal, intrasternal, subcutaneous and intraarticular injection and infusion.

Pharmaceutical compositions for parenteral injection comprise pharmaceutically acceptable sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity may be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption, such as aluminum monostearate and gelatin.

Injectable depot forms are made by forming microencapsule matrices of the drug in biodegradable polymers such as polylactide-polyglycolide, poly(orthoesters), poly(anhydrides), and (poly)glycols, such as PEG. Depending upon the ratio of drug to polymer and the nature of the particular polymer employed, the rate of drug release can be controlled. Depot injectable formulations are also prepared by entrapping the drug in liposomes or microemulsions which are compatible with body tissues.

The injectable formulations may be sterilized, for example, by filtration through a bacterial-retaining filter, or by incorporating sterilizing agents in the form of sterile solid compositions which can be dissolved or dispersed in sterile water or other sterile injectable medium just prior to use.

Topical administration includes administration to the skin or mucosa, including surfaces of the lung and eye. Compositions for topical administration, including those for inhalation, may be prepared as a dry powder which may be pressurized or non-pressurized. In non-pressurized powder compositions, the active ingredient in finely divided form may be used in admixture with a larger-sized pharmaceutically acceptable inert carrier comprising particles having a size, for example, of up to 100 micrometers in diameter. Suitable inert carriers include sugars such as lactose. Desirably, at least 95% by weight of the particles of the active ingredient have an effective particle size in the range of 0.01 to 10 micrometers.

Alternatively, the composition may be pressurized and contain a compressed gas, such as nitrogen or a liquified gas propellant. The liquified propellant medium and indeed the total composition is preferably such that the active ingredient does not dissolve therein to any substantial extent. The pressurized composition may also contain a surface active agent, such as a liquid or solid non-ionic surface active agent or may be a solid anionic surface active agent. It is preferred to use the solid anionic surface active agent in the form of a sodium salt.

A further form of topical administration is to the eye. A compound of the invention is delivered in a pharmaceutically acceptable ophthalmic vehicle, such that the compound is maintained in contact with the ocular surface for a sufficient time period to allow the compound to penetrate the corneal and internal regions of the eye, as for example the anterior chamber, posterior chamber, vitreous body, aqueous humor, vitreous humor, cornea, iris/ciliary, lens, choroid/retina and sclera. The pharmaceutically acceptable ophthalmic vehicle may, for example, be an ointment, vegetable oil or an encapsulating material. Alternatively, the compounds of the invention may be injected directly into the vitreous and aqueous humour.

Compositions for rectal or vaginal administration are preferably suppositories which may be prepared by mixing the compounds of this invention with suitable nonirritating excipients or carriers such as cocoa butter, polyethylene glycol or a suppository wax which are solid at room temperature but liquid at body temperature and therefore melt in the rectum or vaginal cavity and release the active compound.

Compounds of the present invention may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and the phosphatidyl cholines (lecithins), both natural and synthetic. Methods to form liposomes are known in the art. See, for example, Prescott, Ed., Methods in Cell Biology, Volume XIV, Academic Press, New York, N.Y. (1976), p. 33 *et seq.*

While the compounds of the invention can be administered as the sole active pharmaceutical agent, they may also be used in combination with one or more agents which are conventionally administered to patients for treating angiogenic diseases. For example, the compounds of the invention are effective over the short term to make tumors more sensitive to traditional cytotoxic therapies such as chemicals and radiation. The compounds of the invention also enhance the effectiveness of existing cytotoxic adjuvant anti-cancer therapies. The compounds of the invention may also be combined with other antiangiogenic agents to enhance their effectiveness, or combined with other antiangiogenic agents and administered together with other cytotoxic agents. In particular, when used in the treatment of solid tumors, compounds of the invention may be administered with IL-12, retinoids, interferons, angiostatin, endostatin, thalidomide, thrombospondin-1, thrombospondin-2, captopryl, angioinhibins, TNP-470, pentosan polysulfate, platelet factor 4, LM-609, SU-5416, CM-101, Tecogalan, plasminogen-K-5, vasostatin, vitaxin, vasculostatin, squalamine, marimastat or othermMP inhibitors, antineoplastic agents such as alpha inteferon, COMP (cyclophosphamide, vincristine, methotrexate and prednisone), etoposide, mBACOD (methortrexate, bleomycin, doxorubicin, cyclophosphamide, vincristine and dexamethasone), PRO-MACE/MOPP (prednisone, methotrexate (w/leucovin rescue), doxorubicin, cyclophosphamide, cisplatin, taxol, etoposide/mechlorethamine, vincristine, prednisone and procarbazine), vincristine, vinblastine, and the like as well as with radiation.

Total daily dose of the compositions of the invention to be administered to a human or other mammal host in single or divided doses may be in amounts, for example, from 0.0001 to 300 mg/kg body weight daily and more usually 1 to 300 mg/kg body weight.

It will be understood that agents which can be combined with the compound of the present invention for the inhibition, treatment or prophylaxis of angiogenic diseases are not limited to those listed above, but include in principle any agents useful for the treatment or prophylaxis of angiogenic diseases.

The peptides of the invention may be used for the development of affinity columns for isolation of receptors relevant to the antiangiogenic activity of the peptide of the invention, e.g. TSP-1 receptor, in, for example, cultured endothelial cells. Isolation and purification of the receptor may be followed by amino acid sequencing to identify and isolate polynucleotides which encode the receptor. Recombinant expression of this receptor would allow greater amounts of receptor to be produced, e.g. to produce a sufficient quantity for use in high throughput screening assays to identify other angiogenesis inhibitors.

### Determination of Biological Activity

### In Vitro Assay for Angiogenic Activity

The human microvascular endothelial (HMVEC) migration assay was run according to the procedure of S. S. Tolsma, O. V. Volpert, D. J. Good, W. F. Frazier, P. J. Polverini and N. Bouck, J. Cell Biol. 122, 497-511 (1993).

The HMVEC migration assay was carried out using Human Microvascular Endothelial Cells-Dermal (single donor) and Human Microvascular Endothelial Cells, (neonatal). The BCE or HMVEC cells were starved overnight in DME containing 0.1% bovine serum albuminutes (BSA). Cells were then harvested with trypsin and resuspended in DME with 0.1% BSA at a concentration of 1.5 X 10⁶ cells per mL. Cells were added to the bottom of a 48 well modified Boyden chamber (Nucleopore Corporation, Cabin John, MD). The chamber was assembled and inverted, and cells were allowed to attach for 2 hours at 37 °C to polycarbonate chemotaxis membranes (5mM pore size) that had been soaked in 0.1% gelatin overnight and dried. The chamber was then reinverted, and test substances (total volume of 50 µL), including activators, 15 ng/mL bFGF/VEGF, were added to the wells of the upper chamber. The apparatus was incubated for 4 hours at 37 °C. Membranes were recovered, fixed and stained (Diff Quick, Fisher Scientific) and the number of cells that had migrated to the upper chamber per 3 high power fields counted. Background migration to DME + 0.1 BSA was subtracted and the data reported as the number of cells migrated per 10 high power fields (400X) or, when results from multiple experiments were combined, as the percent inhibition of migration compared to a positive control.

Representative compounds described in Examples 1 to 44 inhibited human endothelial cell migration in the above assay by at least 50% inhibition when tested at concentrations of 1 nM. Preferred compounds inhibited human endothelial cell migration by at least 70% when tested at concentrations of 1 nM, and more preferred compounds inhibited human endothelial cell migration by at least 80% at concentrations of 1 nM.

### Synthesis of the Peptides

The polypeptides of the present invention may be synthesized by many techniques that are known to those skilled in the art. For solid phase peptide synthesis, a summary of the many techniques may be found in J.M. Stewart and J.D. Young, Solid Phase Peptide Synthesis, W.H. Freeman Co. (San Francisco), 1963 and J. Meienhofer, Hormonal Proteins and Peptides, vol. 2, p. 46, Academic Press (New York), 1973. For classical solution synthesis see G. Schroder and K. Lupke, The Peptides, vol. 1, Academic Press (New York), 1965.

Reagents, resins, amino acids, and amino acid derivatives are commercially available and can be purchased from Chem-Impex International, Inc. (Wood Dale, IL, U.S.A.) or Calbiochem-Novabiochem Corp. (San Diego, CA, U.S.A.) unless otherwise noted herein.

In general, these methods comprise the sequential addition of one or more amino acids or suitably protected amino acids to a growing peptide chain. Normally, either the amino or carboxy group of the first amino acid is protected by a suitable nitrogen protecting group. The protected or derivatized amino acid can then be either attached to an inert solid support or utilized in solution by adding the next amino acid in the sequence having the complimentary (amino or carboxy) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently to afford the final polypeptide. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide.

A particularly preferred method of preparing compounds of the present invention involves solid phase peptide synthesis.

In this particularly preferred method the α-amino functionality is protected by an acid or base sensitive group. Such protecting groups should have the properties of being stable to the conditions of peptide linkage formation, while being readily removable without destruction of the growing peptide chain or racemization of any of the chiral centers contained therein. Suitable protecting groups are 9-fluorenylmethoxycarbonyl (Fmoc), *t*-butoxycarbonyl (Boc), benzyloxycarbonyl (Cbz), biphenylisopropyl-oxycarbonyl, *t*-amyloxycarbonyl, isobornyloxycarbonyl, (α,α)-dimethyl-3,5-dimethoxybenzyloxycarbonyl, *O*-nitrophenylsulfenyl, 2-cyano-*t*-butoxycarbonyl, and the like. The 9-fluorenylmethoxycarbonyl (Fmoc) protecting group is preferred.

Particularly preferred side chain protecting groups are: for arginine and lysine:
acetyl (Ac), adamantyloxycarbonyl, benzyloxycarbonyl (Cbz), *t*-butoxycarbonyl (Boc), 4-methoxybenzenesulfonyl, N^{G}-4-methoxy-2,3,6-trimethylbenzenesulfonyl (Mtr), 2,2,5,7,8-pentamethylchroman-6-sulfonyl (Pmc), and p-toluenesulfonyl; for asparagine: trityl (Trt); for aspatyl: *t*-butyl (*t*-Bu); for glutamyl: *t*-butyl (*t*-Bu); for glutaminyl: trityl (Trt); for histidine: trityl (Trt), benzyl, benzyloxycarbonyl (Cbz), p-toluenesulfonyl, and 2,4-dinitrophenyl; for penicillamine: methyl; for serine: *t*-butyl (*t*-Bu); for tryptophan: formyl and *t*-butoxycarbonyl (Boc); and for tyrosine: acetyl (Ac), benzyl, *O-*bromobenzyloxycarbonyl, *t*-butyl (*t*-Bu), cyclohexyl, cyclopentyl, 2,6-dichlorobenzyl, and
isopropyl.

In the solid phase peptide synthesis method, the C-terminal amino acid is attached to a suitable solid support or resin. Suitable solid supports useful for the above synthesis are those materials which are inert to the reagents and reaction conditions of the stepwise condensation-deprotection reactions, as well as being insoluble in the media used. The preferred solid support is Seiber ethylamide, which is commercially available from Novabiochem.

The C-terminal amino acid is coupled to the resin by means of a coupling mediatd by N,N'-dicyclohexylcarbodiimide (DCC), N,N'-diisopropylcarbodiimide (DIC), *O-*benzotriazol-1-yl-N,N,N',N'-tetramethyluroniumhexafluorophosphate (HBTU), or [*O*-(7-azabenzotriazol-1-yl)-1,1,3,3-tetramethyluronium hexafluorophosphate] (HATU), with or without 4-dimethylaminopyridine (DMAP), 1-hydroxybenzotriazole (HOBT), benzotriazol-1-yloxy-tris(dimethylamino)phosphoniumhexafluorophosphate (BOP), or bis(2-oxo-3-oxazolidinyl)phosphine chloride (BOPCI), for about 1 to about 24 hours at a temperature between 10 °C and 50 °C in a solvent such as dichloromethane or DMF. The Fmoc group is cleaved with a secondary amine, preferably piperidine, prior to coupling with the C-terminal amino acid as described above. The preferred method for coupling to the deprotected 4-(2',4'-dimethoxyphenyl-Fmoc-aminomethyl)phenoxyacetamidoethyl resins are *O*-benzotriazol-1-yl-N,N,N',N'-tetramethyluroniumhexafluorophosphate (HBTU, 1 equiv.) and 1-hydroxybenzotriazole (HOBT, 1 equiv.), or [*O*-(7-azabenzotriazol-1-yl)-1,1,3,3-tetramethyluronium hexafluorophosphate] (HATU, 1 equiv.) in DMF.

The coupling of successive protected amino acids can be carried out in an automatic polypeptide synthesizer as is well known in the art. In a preferred embodiment, the α-amino function in the amino acids of the growing peptide chain are protected with Fmoc. The removal of the Fmoc protecting group from the N-terminal side of the growing peptide is accomplished by treatment with a secondary amine, preferably piperidine. Each protected amino acid is then introduced in about 3-fold molar excess and the coupling is preferably carried out in DMF. The coupling agent is normally *O*-benzotriazol-1-yl-N,N,N',N'-tetramethyluroniumhexafluorophosphate (HBTU, 1 equiv.) and 1-hydroxybenzotriazole (HOBT, 1 equiv.) or [*O*-(7-azabenzotriazol-1-yl)-1,1,3,3-tetramethyluronium hexafluorophosphate] (HATU, 1 equiv.).

At the end of the solid phase synthesis, the polypeptide is removed from the resin and deprotected, either in succession or in a single operation. Removal of the polypeptide and deprotection can be accomplished in a single operation by treating the resin-bound polypeptide with a cleavage reagent, for example trifluoroacetic acid containing thioanisole, water, or ethanedithiol.

In cases wherein the C-terminus of the polypeptide is an alkylamide, the resin is cleaved by aminolysis with an alkylamine. Alternatively, the peptide may be removed by transesterification, e.g. with methanol, followed by aminolysis or by direct transamidation. The protected peptide may be purified at this point or taken to the next step directly. The removal of the side chain protecting groups is accomplished using the cleavage cocktail described above.

The fully deprotected peptide is purified by a sequence of chromatographic steps employing any or all of the following types: ion exchange on a weakly basic resin in the acetate form; hydrophobic adsorption chromatography on underivitized polystyrene-divinylbenzene (for example, AMBERLITE® XAD); silica gel adsorption chromatography; ion exchange chromatography on carboxymethylcellulose; partition chromatography, e.g. on SEPHADEX® G-25, LH-20 or countercurrent distribution; high performance liquid chromatography (HPLC), especially reverse-phase HPLC on octyl- or octadecylsilyl-silica bonded phase column packing.

Abbreviations which have been used the following examples are: NMP for N-methylpyrrolidinone; HBTU for 2-(1H-benzotriazole-1-yl)-1,1,3,3-tetramethyluronium hexafluorophosphate; DMF for N,N-dimethylformamide, and TFA for trifluoroacetic acid.

### EXAMPLE 1

### N-Ac-Sar-Gly-Lys(Ac)-D-Leu-Thr-Nva-Ile-Arg-ProNH-ethyl

In the reaction vessel of an Applied Biosystems 433A peptide synthesizer was placed 0.1 mM of Fmoc-Pro-Sieber ethylamide resin. Cartridges of 1mM amino acids were sequentially loaded. The Fastmoc 0.1 with previous peak monitoring protocol was used with the following is the synthetic cycle:
1. Resin solvated with NMP for about 5 minutes;
2. Resin washed with NMP for about 5 minutes;
3. Fmoc group removed using 50% piperidine solution in NMP for 5 minutes, resin washed, and the sequence repeated 3 to 4 times;
4. Fmoc-amino acid activated with 1mM of 0.5M HBTU in DMF;
5. Activated Fmoc-amino acid added to the reaction vessel followed by addition of 1mM of 2M diisopropylamine in NMP solution;
6. Fmoc-amino acid coupled for 20 minutes;
7. Resin washed and Fmoc-group removed using 50% piperidine in NMP.
The following protected amino acids were sequentially coupled to the resin using above protocol:

| Amino acid | Coupling time |
|---|---|
| 1. Fmoc-Arg(Pmc) | 20 minutes |
| 2. Fmoc-Ile | 20 minutes |
| 3. Fmoc-Nva | 20 minutes |
| 4. Fmoc-Thr(*t*-Bu) | 20 minutes |
| 5. Fmoc-D-Leu | 20 minutes |
| 6. Fmoc-Lys(Ac) | 20 minutes |
| 7. Fmoc-Gly | 20 minutes |
| 8. Fmoc-Sar | 20 minutes |
| 9. acetic acid | 20 minutes |

Upon completion of the synthesis the resin-bound peptide was washed with methanal three times and dried *in vacuo*, then treated with a (95:5) TFA/water solution (3 mL) at room temperature overnight. The resin was filtered and washed 3 times with methanol. The filtrates and the washes were combined and concentrated *in vacuo.* The residue was treated with ether and the precipitate was filtered to provide the crude peptide as an amorphous powder. This was purified by preparative HPLC using a C-18 column with a solvent system increasing in gradient from 5% to 100% acetonitrile/water containing 0.1 % TFA over a period of 50 minutes. The pure fractions were lyophilized to provide N-Ac-Sar-Gly-Lys(Ac)-D-Leu-Thr-Nva-Ild-Arg-ProNH-ethyl as the trifluoroacetate salt; Rₜ = 2.764 minutes (using a C-18 column and a solvent system increasing in gradient from 20% to 95% acetonitrile/water containing 10mM ammonium acetate over a period of 10 minutes); MS (ESI) m/e 1065 (M+H).

### EXAMPLE 2

### N-Ac-Sar-Gly-5-BrThiAla-D-Leu-Thr-Nva-Ile-Arg-ProNH-ethyl

The desired product was prepared by substituting Fmoc-5-BrThiAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-5-BrThiAla-D-Leu-Thr-Nva-Ile-Arg-ProNH-ethyl as the trifluoroacetate salt; Rₜ = 4.392 minutes (using a C-18 column and a solvent system increasing in gradient from 20% to 95% acetonitrile/water containing 10mM ammonium acetate over a period of 10 minutes); MS (ESI) m/e 1128 (M+H).

### EXAMPLE 3

### N-Ac-Sar-Gly-3-CNPheAla-D-Leu-Thr-Nva-Ile-Arg-ProNH-ethyl

The desired product was prepared by substituting Fmoc-3CNPheAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-3-CNPheAla-D-Leu-Thr-Nva-Ile-Arg-ProNH-ethyl as the trifluoroacetate salt; Rₜ = 3.805 minutes (using a C-18 column and a solvent system increasing in gradient from 20% to 95% acetonitrile/water containing 10mM ammonium acetate over a period of 10 minutes); MS (ESI)m/e 1065 (M⁺).

### EXAMPLES 4

### N-Ac-Sar-Gly-Cys(Et)-D-Leu-Thr-Nva-Ile-Arg-ProNH-ethyl

The desired product was prepared by substituting Fmoc-Cys(Et) for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-Cys(Et)-D-Leu-Thr-Nva-Ile-Arg-ProNH-ethyl as the trifluoroacetate salt; Rₜ = 3.629 minutes (using a C-18 column and a solvent system increasing in gradient from 20% to 95% acetonitrile/water containing 10mM ammonium acetate over a period of 10 minutes); MS (ESI) m/e 1024 (M⁺).

### EXAMPLE 5

### N-Ac-Sar-Gly-3-ThzAla-D-Leu-Thr-Nva-Ile-Arg-ProNH-ethyl

The desired product was prepared by substituting Fmoc-3-ThzAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-3-ThzAla-D-Leu-Thr-Nva-Ile-Arg-ProNH-ethyl as the trifluoroacetate salt; Rₜ = 3.172 minutes (using a C-18 column and a solvent system increasing in gradient from 20% to 95% acetonitrile/water containing 10mM ammonium acetate over a period of 10 minutes); MS (ESI) m/e 1049 (M⁺).

### EXAMPLE 6

### N-Ac-Sar-Gly-(1R,4S)-AmCyeCO-D-Leu-Thr-Nva-Ile-Arg-ProNH-ethyl

The desired product was prepared by substituting (1R,4S)-1-Fmoc-amino-cyclopent-2-ene-4-carboxylic acid for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-(1R,4S)-AmCyeCO-D-Leu-Thr-Nva-Ile-Arg-ProNH-ethyl as the trifluoroacetate salt; Rₜ = 2.961 minutes (using a C-18 column and a solvent system increasing in gradient from 20% to 95% acetonitrile/water containing 10mM ammonium acetate over a period of 10 minutes); MS (ESI) m/e 1004 (M⁺).

### EXAMPLE 7

### N-Ac-Sar-Gly-3,4-diOMePheAla-D-Leu-Thr-Nva-Ile-Arg-ProNH-ethyl

The desired product was prepared by substituting Fmoc-3,4-diOMePheAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-3,4-diOMePheAla-D-Leu-Thr-Nva-Ile-Arg-ProNH-ethyl as the trifluoroacetate salt; Rₜ = 3.49 minutes (using a C-18 column and a solvent system increasing in gradient from 20% to 95% acetonitrile/water containing 10mM ammonium acetate over a period of 10 minutes); MS (ESI) m/e 1102 (M⁺).

### EXAMPLE 8

### N-Ac-Sar-Gly-4-MePheAla-D-Leu-Thr-Nva-Ile-Arg-ProNH-ethyl

The desired product was prepared by substituting Fmoc-4-MePheAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-4-MePheAla-D-Leu-Thr-Nva-Ile-Arg-ProNH-ethyl as the trifluoroacetate salt; Rₜ = 4.2 minutes (using a C-18 column and a solvent system increasing in gradient from 20% to 95% acetonitrile/water containing 10mM ammonium acetate over a period of 10 minutes); MS (ESI) m/e 1056 (M⁺).

### EXAMPLE 9

### N-Ac-Sar-Gly-3-CiPheAla-D-Leu-Thr-Nva-Ile-Arg-ProNH-ethyl

The desired product was prepared by substituting Fmoc-3-CIPheAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-3-ClPheAla-D-Leu-Thr-Nva-Ile-Arg-ProNH-ethyl as the trifluoroacetate salt; Rₜ = 4.366 minutes (using a C-18 column and a solvent system increasing in gradient from 20% to 95% acetonitrile/water containing 10mM ammonium acetate over a period of 10 minutes); MS (ESI) m/e 1076 (M⁺).

### EXAMPLE 10

### N-Ac-Sar-Gly-2-ThiAla-D-Leu-Thr-Nva-Ile-Arg-ProNH-ethyl

The desired product was prepared by substituting Fmoc-3-(thien-2-yl)alanine for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-2-ThiAla-D-Leu-Thr-Nva-Ile-Arg-ProNH-ethyl as the trifluoroacetate salt; Rₜ = 3.826 minutes (using a C-18 column and a solvent system increasing in gradient from 20% to 95% acetonitrile/water containing 10mM ammonium acetate over a period of 10 minutes); MS (ESI) m/e 1046 (M⁺).

### EXAMPLE 11

### N-Ac-Sar-Gly-PheGly-D-Leu-Thr-Nva-Ile-Arg-ProNH-ethyl

The desired product was prepared by substituting Fmoc-PheGly for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-PheGly-D-Leu-Thr-Nva-Ile-Arg-ProNH-ethyl as the trifluoroacetate salt; Rₜ = 3.73 minutes (using a C-18 column and a solvent system increasing in gradient from 20% to 95% acetonitrile/water containing 10mM ammonium acetate over a period of 10 minutes); MS (ESI) m/e 1026 (M⁺).

### EXAMPLE 12

### N-Ac-Sar-Gly-2 4-Diabu-D-Leu-Thr-Nva-Ile-Arg-ProNH-ethyl

The desired product was prepared by substituting 4-Boc-amino-2- Fmoc-butanoic acid for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-2,4-Diabu-D-Leu-Thr-Nva-Ile-Arg-ProNH-ethyl as the trifluoroacetate salt; Rₜ = 2.50 minutes (using a C-18 column and a solvent system increasing in gradient from 20% to 95% acetonitrile/water containing 10mM ammonium acetate over a period of 10 minutes); MS (ESI) m/e 995 (M⁺).

### EXAMPLE 13

### N-Ac-Sar-Gly-Met(O₂)-D-Leu-Thr-Nva-Ile-Arg-ProNH-ethyl

The desired product was prepared by substituting Fmoc-Met(O₂) for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-Met(O₂)-D-Leu-Thr-Nva-Ile-Arg-ProNH-ethyl as the trifluoroacetate salt; Rₜ = 2.874 minutes (using a C-18 column and a solvent system increasing in gradient from 20% to 95% acetonitrile/water containing 10mM ammonium acetate over a period of 10 minutes); MS (ESI) m/e 1058 (M⁺).

### EXAMPLE 14

### N-Ac-Sar-Gly-2-Nal-D-Leu-Thr-Nva-Ile-Arg-ProNH-ethyl

The desired product was prepared by substituting Fmoc-2-Nal for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-2-Nal-D-Leu-Thr-Nva-Ile-Arg-ProNH-ethyl as the trifluoroacetate salt; Rₜ = 4.56 minutes (using a C-18 column and a solvent system increasing in gradient from 20% to 95% acetonitrile/water containing 10mM ammonium acetate over a period of 10 minutes); MS (ESI) m/e 1092 (M⁺).

### EXAMPLE 15

### N-Ac-Sar-Gly-1-Nal-D-Leu-Thr-Nva-Ile-Arg-ProNH-ethyl

The desired product was prepared by substituting Fmoc-1-Nal for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-1-Nal-D-Leu-Thr-Nva-Ile-Arg-ProNH-ethyl as the trifluoroacetate salt; Rₜ = 4.558 minutes (using a C-18 column and a solvent system increasing in gradient from 20% to 95% acetonitrile/water containing 10mM ammonium acetate over a period of 10 minutes); MS (ESI) m/e 1092 (M⁺).

### EXAMPLE 16

### N-Ac-Sar-Gly-2-Abu-D-Leu-Thr-Nva-Ile-Arg-ProNH-ethyl

The desired product was prepared by substituting Fmoc-2-Abu for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-2-Abu-D-Leu-Thr-Nva-Ile-Arg-ProNH-ethyl as the trifluoroacetate salt; Rₜ = 3.181 minutes (using a C-18 column and a solvent system increasing in gradient from 20% to 95% acetonitrile/water containing 10mM ammonium acetate over a period of 10 minutes); MS (ESI) m/e 980 (M⁺).

### EXAMPLE 17

### N-Ac-Sar-Gly-Met(O)-D-Leu-Thr-Nva-Ile-Arg-ProNH-ethyl

The desired product was prepared by substituting Fmoc-Met(O) for Fmoc-Lys(Ac) in Exmaple 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-Met(O)-D-Leu-Thr-Nva-Ile-Arg-ProNH-ethyl as the trifluoroacetate salt; Rₜ = 2.543 minutes (using a C-18 column and a solvent system increasing in gradient from 20% to 95% acetonitrile/water containing 10mM ammonium acetate over a period of 10 minutes); MS (ESI) m/e 1042 (M⁺).

### EXAMPLE 18

### N-Ac-Sar-Gly-Orn-D-Leu-Thr-Nva-Ile-Arg-ProNH-ethyl

The desired product was prepared by substituting Fmoc-α-amino-Boe-δ-amino-Om for Fmoc-Lys(Ac) in Example 1. Upon completion of the synthesis; cleavage of the peptide from the resin, removal of the protecting groups, precipitation with diethyl ether, and filtration, the crude peptide was obtained. This was purified by preparative HPLC to provide N-Ac-Sar-Gly-Orn-D-Leu-Thr-Nva-Ile-Arg-ProNH-ethyl as the trifluoroacetate salt; Rₜ = 2.455 minutes (using a C-18 column and a solvent system increasing in gradient from 20% to 95% acetonitrile/water containing 10mM ammonium acetate over a period of 10 minutes); MS (ESI) m/e 1009 (M⁺).

### EXAMPLE 19

### N-Ac-Sar-Gly-4-ClPheAla-D-Leu-Thr-Nva-Ile-Arg-ProNH-ethyl

The desired product was prepared by substituting Fmoc-4-ClPheAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-4-ClPheAla-D-Leu-Thr-Nva-Ile-Arg-ProNH-ethyl as the trifluoroacetate salt; Rₜ = 4.089 minutes (using a C-18 column and a solvent system increasing in gradient from 20% to 95% acetonitrile/water containing 10mM ammonium acetate over a period of 10 minutes); MS (ESI) m/e 1076 (M⁺).

### EXAMPLE 20

### N-Ac-Sar-Gly-HPheAla-D-Leu-Thr-Nva-Ile-Arg-ProNH-ethyl

The desired product was prepared by substituting Fmoc-HPheAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-HPheAla-D-Leu-Thr-Nva-Ile-Arg-ProNH-ethyl as the trifluoroacetate salt; Rₜ = 4.265 minutes (using a C-18 column and a solvent system increasing in gradient from 20% to 95% acetonitrile/water containing 10mM ammonium acetate over a period of 10 minutes); MS (ESI) m/e 1056 (M⁺).

### EXAMPLE 21

### N-Ac-Sar-Gly-Tic-D-Leu-Thr-Nva-Ile-Arg-ProNH-ethyl

The desired product was prepared by substituting Fmoc-Tic for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-Tic-D-Leu-Thr-Nva-Ile-Arg-ProNH-ethyl as the trifluoroacetate salt; Rₜ = 4.155 minutes (using a C-18 column and a solvent system increasing in gradient from 20% to 95% acetonitrile/water containing 10mM ammonium acetate over a period of 10 minutes); MS (ESI) m/e 1054 (M⁺).

### EXAMPLE 22

### N-Ac-Sar-Gly-StyAla-D-Leu-Thr-Nva-Ile-Arg-ProNH-ethyl

The desired product was prepared by substituting Fmoc-StyAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-StyAla-D-Leu-Thr-Nva-Ile-Arg-ProNH-ethyl as the trifluoroacetate salt; Rₜ = 4.475 minutes (using a C-18 column and a solvent system increasing in gradient from 20% to 95% acetonitrile/water containing 10mM ammonium acetate over a period of 10 minutes); MS (ESI) m/e 1068 (M⁺).

### EXAMPLE 23

### N-Ac-Sar-Gly-Cys(Me)-D-Leu-Thr-Nva-Ile-Arg-ProNH-ethyl

The desired product was prepared by substituting Fmoc-Cys(Me) for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-Cys(Me)-D-Leu-Thr-Nva-Ile-Arg-ProNH-ethyl as the trifluoroacetate salt; Rₜ = 4.045 minutes (using a C-18 column and a solvent system increasing in gradient from 20% to 95% acetonitrile/water containing 10mM ammonium acetate over a period of 10 minutes); MS (ESI) m/e 1087 (M⁺).

### EXAMPLE 24

### N-Ac-Sar-Gly-AllylGly-D-Leu-Thr-Nva-Ile-Arg-ProNH-ethyl

The desired product was prepared by substituting Fmoc-AllylGly for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-AllylGly-D-Leu-Thr-Nva-Ile-Arg-ProNH-ethyl as the trifluoroacetate salt; Rₜ = 3.33 minutes (using a C-18 column and a solvent system increasing in gradient from 20% to 95% acetonitrile/water containing 10mM ammonium acetate over a period of 10 minutes); MS (ESI) m/e 992 (M⁺).

### EXAMPLE 25

### N-Ac-Sar-Gly-Cys(Et)-D-Leu-Thr-Nva-Ile-Arg-ProNH-ethyl

The desired product was prepared by substituting Fmoc-Cys(Et) for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-Cys(Et)-D-Leu-Thr-Nva-Ile-Arg-ProNH-ethyl as the trifluoroacetate salt; Rₜ = 3.629 minutes (using a C-18 column and a solvent system increasing in gradient from 20% to 95% acetonitrile/water containing 10mM ammonium acetate over a period of 10 minutes); MS (ESI) m/e 1026 (M⁺).

### EXAMPLES 26

### N-Ac-Sar-Gly-4-FPheAla-D-Leu-Thr-Nva-Ile-Arg-ProNH-ethyl

The desired product was prepared by substituting Fmoc-4-FPheAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-4-FPheAla-D-Leu-Thr-Nva-Ile-Arg-ProNH-ethyl as the trifluoroacetate salt; Rₜ = 4.053 minutes (using a C-18 column and a solvent system increasing in gradient from 20% to 95% acetonitrile/water containing 10mM ammonium acetate over a period of 10 minutes); MS (ESI) m/e 1060 (M⁺).

### EXAMPLE 27

### N-Ac-Sar-Gly-2,3-Diapr-D-Leu-Thr-Nva-Ile-Arg-ProNH-ethyl

The desired product was prepared by substituting Fmoc-α-amino-Boc-β-aminopropionic acid for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-2,3-Diapr-D-Leu-Thr-Nva-Ile-Arg-ProNH-ethyl as the trifluoroacetate salt; Rₜ = 2.529 minutes (using a C-18 column and a solvent system increasing in gradient from 20% to 95% acetonitrile/water containing 10mM ammonium acetate over a period of 10 minutes); MS (ESI) m/e 981 (M⁺).

### EXAMPLE 28

### N-Ac-Sar-Gly-Met(O₂)-D-Ile-Thr-Nva-Ile-Arg-ProNH-ethyl

The desired product was prepared by substituting Fmoc-Met(O₂) for Fmoc-Lys(Ac) and Fmoc-D-Ile for Fmoc-D-Leu in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-Met(O₂)-D-Ile-Thr-Nva-Ile-Arg-ProNH-ethyl as the trifluoroacetate salt; Rₜ = 2.64 minutes (using a C-18 column and a solvent system increasing in gradient from 20% to 95% acetonitrile/water containing 10mM ammonium acetate over a period of 10 minutes); MS (ESI) m/e 1058 (M+H); Amino Acid Anal.: 0.98 Sar; 0.94 Gly; 0.90 Met(O₂); 2.04 Ile; 0.59 Thr; 0.97 Nva; 1.32 Arg; 1.07 Pro.

### EXAMPLE 29

### N-Ac-Sar-Gly-3-Pal-D-Ile-Thr-Nva-Ile-Arp:-ProNH-ethyl

The desired product was prepared by substituting Fmoc-3-Pal for Fmoc-Lys(Ac) and Fmoc-D-Ile for Fmoc-D-Leu in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-3-Pal-D-Ile-Thr-Nva-Ile-Arg-ProNH-ethyl as the trifluoroacetate salt; Rₜ = 2.79 minutes (using a C-18 column and a solvent system increasing in gradient from 20% to 95% acetonitrfle/water containing 10mM ammonium acetate over a period of 10 minutes); MS (ESI) m/e 1043 (M+H); Amino Acid Anal.: 1.00 Sar; 0.95 Gly; 0.89 3Pal; 2.05 Ile; 0.55 Thr; 0.99 Nva; 1.43 Arg; 1.08 Pro.

### EXAMPLE 30

### N-Ac-Sar-Gly-4-ClPheAla-D-Ile-Thr-Nva-Ile-Arg-ProNH-ethyl

The desired product was prepared by substituting Fmoc-4-ClPheAla for Fmoc-Lys(Ac) and Fmoc-D-Ile for Fmoc-D-Leu in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-4-ClPheAla-D-Ile-Thr-Nva-Ile-Arg-ProNH-ethyl as the trifluoroacetate salt; Rₜ = 4.17 minutes (using a C-18 column and a solvent system increasing in gradient from 20% to 95% acetonitrile/water containing 10mM ammonium acetate over a period of 10 minutes); MS (ESI) m/e 1076 (M+H); Amino Acid Anal.: 0.97 Sar; 0.96 Gly; 1.20 4ClPheAla; 2.08 Ile; 0.49 Thr; 0.96 Nva; 1.39 Arg; 1.07 Pro.

### EXAMPLE 31

### N-Ac-Sar-Gly-1-Nal-D-Ile-Thr-Nva-Ile-Arg-ProNH-ethyl

The desired product was prepared by substituting Fmoc-1-Nal for Fmoc-Lys(Ac) and Fmoc-D-Ile for Fmoc-D-Leu in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-1-Nal-D-Ile-Thr-Nva-Ile-Arg-ProNH-ethyl as the trifluoroacetate salt; Rₜ = 4.34 minutes (using a C-18 column and a solvent system increasing in gradient from 20% to 95% acetonitrile/water containing 10mM ammonium acetate over a period of 10 minutes); MS (ESI) m/e 1092 (M+H); Amino Acid Anal.: 1.06 Sar; 0.96 Gly; 2.09 Ile; 0.48 Thr; 1.00 Nva; 1.41 Arg; 1.01 Pro.

### EXAMPLE 32

### N-Ac-Sar-Gly-2-Nal-D-Ile-Thr-Nva-Ile-Ar-PrONH-ethyl

The desired product was prepared by substituting Fmoc-2-Nal for Fmoc-Lys(Ac) and Fmoc-D-Ile for Fmoc-D-Leu in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-2-Nal-D-Ile-Thr-Nva-Ile-Arg-ProNH-ethyl as the trifluoroacetate salt; Rₜ = 4.36 minutes (using a C-18 column and a solvent system increasing in gradient from 20% to 95% acetonitrile/water containing 10mM ammonium acetate over a period of 10 minutes); MS (ESI) m/e 1092 (M+H); Amino Acid Anal.: 0.94 Sar; 0.97 Gly; 2.05 Ile; 0.56 Thr; 0.97 Nva; 1.38 Arg; 1.08 Pro.

### EXAMPLE 33

### N-Ac-Sar-Gly-3-FPheAla-D-Ile-Thr-Nva-Ile-Are-ProNH-ethyl

The desired product was prepared by substituting Fmoc-3FPheAla for Fmoc-Lys(Ac) and Fmoc-D-Ile for Fmoc-D-Leu in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-3-FPheAla-D-Ile-Thr-Nva-Ile-Arg-ProNH-cthyl as the trifluoroacetate salt; Rₜ = 3.78 minutes (using a C-18 column and a solvent system increasing in gradient from 20% to 95% acetonitrile/water containing 10mM ammonium acetate over a period of 10 minutes); MS (ESI) m/e 1060 (M+H); Amino Acid Anal.: 1.06 Sar; 0.96 Gly; 1.04 3FPheAla; 2.01 Ile; 0.47 Thr; 1.00 Nva; 1.41 Arg; 1.03 Pro.

### EXAMPLE 34

### N-Ac-Sar-Gly-HPheAla-D-Ile-Thr-Nva-Ile-Arg-ProNH-ethyl

The desired product was prepared by substituting Fmoc-HPheAla for Fmoc-Lys(Ac) and Fmoc-D-Ile for Fmoc-D-Leu in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-HPheAla-D-Ile-Thr-Nva-Ile-Arg-ProNH-ethyl as the trifluoroacetate salt; Rₜ = 3.70 minutes (using a C-18 column and a solvent system increasing in gradient from 20% to 95% acetonitrile/water containing 10mM ammonium acetate over a period of 10 minutes); MS (ESI) m/e 1056 (M+H); Amino Acid Anal.: 1.01 Sar; 0.97 Gly; 1.09 HPheAla; 2.11 Ile; 0.49 Thr; 1.03 Nva; 1.39 Arg; 1.09Pro.

### EXAMPLE 35

### N-Ac-Sar-Gly-4-FPheAla-D-Ile-Thr-Nva-Ile-Arg-ProNH-ethyl

The desired product was prepared by substituting Fmoc-4-FPheAla for Fmoc-Lys(Ac) and Fmoc-D-Ile for Fmoc-D-Leu in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-4-FPheAla-D-Ile-Thr-Nva-Ile-Arg-ProNH-ethyl as the trifluoroacetate salt; Rₜ = 3.89 minutes (using a C-18 column and a solvent system increasing in gradient from 20% to 95% acetonitrile/water containing 10mM ammonium acetate over a period of 10 minutes); MS (ESI) m/e 1060 (M+H); Amino Acid Anal.: 0.97 Sar; 0.97 Gly; 0.46 4FPheAla; 1.73 Ile; 0.49 Thr; 1.01 Nva; 1.45 Arg; 1.03 Pro.

### EXAMPLE 36

### N-Ac-Sar-Gly-alloIle-D-Ile-Thr-Nva-Ile-Arg-ProNH-ethyl

The desired product was prepared by substituting Fmoc-alloIle for Fmoc-Lys(Ac) and Fmoc-D-Ile for Fmoc-D-Leu in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-alloIle-D-Ile-Thr-Nva-Ile-Arg-ProNH-ethyl as the trifluoroacetate salt; Rₜ = 3.44 minutes (using a C-18 column and a solvent system increasing in gradient from 20% to 95% acetonitrile/water containing 10mM ammonium acetate over a period of 10 minutes); MS (ESI) m/e 1008 (M+H); Amino Acid Anal.: 1.08 Sar; 0.98 Gly; 2.99 Ile; 0.54 Thr; 1.02 Nva; 1.45 Arg; 1.02 Pro.

### EXAMPLE 37

### N-Ac-Sar-Gly-Ser(Bzl)-D-Leu-Thr-Nva-Ile-Arg-ProNH-ethyl

The desired product was prepared by substituting Fmoc-Ser(Bzl) for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-Ser(Bzl)-D-Leu-Thr-Nva-Ile-Arg-ProNH-ethyl as the trifluoroacetate salt; Rₜ = 4.171 minutes (using a C-18 column and a solvent system increasing in gradient from 20% to 95% acetonitrile/water containing 10mM ammonium acetate over a period of 10 minutes); MS (ESI) m/e 1070 (M⁺).

### EXAMPLE 38

### N-Ac-Sar-Gly-HSer-D-Ile-Thr-Nva-Ile-Arg-ProNH-ethyl

The desired product was prepared by substituting Fmoc-HSer(*t*-Bu) for Fmoc-Lys(Ac) and Fmoc-D-Ile for Fmoc-D-Leu in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-HSer-D-Ile-Thr-Nva-Ile-Arg-ProNH-ethyl as the trifluoroacetate salt; Rₜ = 2.4 minutes (using a C-18 column and a solvent system increasing in gradient from 20% to 95% acetonitrile/water containing 10mM ammonium acetate over a period of 10 minutes); MS (ESI) m/e 996 (M+H); Amino Acid Anal.: 1.01 Sar; 0.97 Gly; 0.43 HSer; 2.03 Ile; 0.55 Thr; 0.94 Nva; 1.31 Arg; 1.04 Pro.

### EXAMPLE 39

### N-Ac-Sar-Gly-(1R,4S)-AmCyeCO-D-Leu-Ser-Ser-Ile-Arg-ProNH-ethyl

The desired product was prepared by substituting Fmoc-(1R,4S)-AmCyeCO for Fmoc-Lys(Ac), Fmoc-Ser(t-Bu) for Fmoc-Thr(*t*-Bu) and Fmoc-Ser(*t*-Bu) for Fmoc-Nva in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-(1R,4S)-AmCyeCO-D-Leu-Ser-Ser-Ile-Arg-ProNH-ethyl as the trifluoroacetate salt; Rₜ = 2.69 minutes (using a C-18 column and a solvent system increasing in gradient from 20% to 95% acetonitrile/water containing 10mM ammonium acetate over a period of 10 minutes); MS (ESI) m/e 978 (M+H)⁺; Amino Acid Anal.: 1.02 Sar; 0.96 Gly; 1.04 Leu; 1.01 Ile; 0.79 Ser; 1.00 Arg; 1.03 Pro.

### EXAMPLE 40

### N-(6-MeNicotinyl)-Sar-Gly-(1R,4S)-AmCyeCO-D-Leu-Thr-Nva-Ile-Arg-ProNH-ethyl

The desired product was prepared by substituting 6-methylnicotinic acid for acetic acid and Fmoc-(1R,4S)-AmCyeCO for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-(6-MeNicotinyl)-Sar-Gly-(1R,4S)-AmCyeCO-D-Leu-Thr-Nva-Ile-Arg-ProNH-ethyl as the trifluoroacetate salt; Rₜ = 3.67 minutes (using a C-18 column and a solvent system increasing in gradient from 20% to 95% acetonitrile/water containing 10mM ammonium acetate over a period of 10 minutes); MS (ESI) m/e 1081 (M+H)⁺; Amino Acid Anal.: 0.98 Sar; 0.97 Gly; 1.02 Leu; 0.51 Thr; 1.03 Nva; 1.01 Ile; 1.05 Arg; 1.02 Pro.

### EXAMPLE 41

### N-Ac-Sar-Gly-D-ThiAla-D-Leu-Thr-Nva-Ile-Arg-ProNH-ethyl

The desired product was prepared by substituting Fmoc-D-ThiAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-D-ThiAla-D-Leu-Thr-Nva-Ile-Arg-ProNH-ethyl as the trifluoroacetate salt; Rₜ = 4.03 minutes (using a C-18 column and a solvent system increasing in gradient from 20% to 95% acetonitrile/water containing 10mM ammonium acetate over a period of 10 minutes); MS (ESI) m/e 1048 (M+H)⁺; Amino Acid Anal.: 0.99 Sar; 0.94 Gly; 1.02 Leu; 0.45 Thr; 0.99 Nva; 1.03 Ile; 1.01 Arg; 1.00 Pro.

### EXAMPLE 42

### N-Ac-Sar-Gly-3CNPheAla-D-Leu-Thr-Nva-Ile-Arg-Pro-DAlaNH₂

The desired product was prepared by substituting Fmoc-DAla-Sieber amide resin for Fmoc-Pro-Sieber ethylamide resin and Fmoc-3CNPheAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-3CNPheAla-DLeu-Thr-Nva-Ile-Arg-ProNH-ethyl as the trifluoroacetate salt; Rₜ = 3.83 minutes (using a C-18 column and a solvent system increasing in gradient from 20% to 95% acetonitrile/water containing 1 0mM ammonium acetate over a period of 10 minutes); MS (ESI) m/e 1110 (M+H)⁺; Amino Acid Anal.: 1.02 Sar; 0.98 Gly; 0.96 Leu; 0.48 Thr; 1.01 Nva; 0.99 Ile; 1.04 Arg; 1.1 Pro; 1.03 Ala.

### EXAMPLE 43

### N-Ac-Sar-Gly-ThiAla-D-Leu-Thr-Nva-Ile-Arg-Pro-D-AlaNH₂

The desired product was prepared by substituting Fmoc-DAla-Sieber amide resin for Fmoc-Pro-Sieber ethylamide resin and Fmoc-ThiAla for Fmoc-Lys(Ac) in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-ThiAla-D-Leu-Thr-Nva-Ile-Arg-Pro-DAlaNH₂ as the trifluoroacetate salt; Rₜ = 3.77 minutes (using a C-18 column and a solvent system increasing in gradient from 20% to 95% acetonitrile/water containing 10mM ammonium acetate over a period of 10 minutes); MS (ESI) m/e 1091 (M+H)⁺; Amino Acid Anal.: 0.96 Sar; 1.03 Gly; 1.02 Leu; 0.43 Thr; 1.02 Nva; 0.97 Ile; 1.00 Arg; 1.08 Pro.

### EXAMPLE 44

### N-Ac-Sar-Gly-(1R,4S)-AmCyeCO-D-Leu-Thr-Gln-Ile-Arg-ProNH-ethyl

The desired product was prepared by substituting Fmoc-(1R,4S)-AmCyeCO for Fmoc-Lys(Ac) and Fmoc-Gln(Trt) for Fmoc-Nva in Example 1. Upon completion of the synthesis, cleavage of the peptide from the resin, removal of the protecting groups, precipitation with diethyl ether, and filtration, the crude peptide was obtained. This was purified by preparative HPLC to provide N-Ac-Sar-Gly-(1R,4S)-AmCyeCO-D-Leu-Thr-Gln-Ile-Arg-ProNH-ethyl as the trifluoroacetate salt; Rₜ = 2.78 minutes (using a C-18 column and a solvent system increasing in gradient from 20% to 95% acetonitrile/water containing 10mM ammonium acetate over a period of 10 minutes); MS (ESI) m/e 1033 (M⁺); Amino Acid Anal.: 1.00 Sar; 1.02 Gly; 1.08 Leu; 0.58 Thr; 0.98 Glu; 1.01 Ile; 1.00 Arg; 1.01 Pro.

## Claims

1. A compound of formula (I) or a pharmaceutically acceptable salt thereof, wherein
Xaa₁ is selected from the group consisting of
acetyl, and
6-methylnicotinyl;
Xaa₂ is sarcosyl;
Xaa₃ is glycyl;
Xaa₄ is selected from the group consisting of
alloisoleucyl,
allylglycyl,
2-aminobutyryl,
(1R,4S)-1-aminocyclopent-2-ene-4-carbonyl,
3-(5-bromothien-2-yl)alanyl,
3-(3-chlorophenyl)alanyl,
3-(4-chlorophenyl)alanyl,
3-(3-cyanophenyl)alanyl,
cysteinyl(S-ethyl),
cysteinyl(S-methyl),
2,3-diaminopropionyl,
2,4-diaminobutanoyl,
3-(3,4-dimethoxyphenyl)alanyl,
3-(3-fluorophenyl)alanyl,
3-(4-fluorophenyl)alanyl,
homophenylalanyl,
homoseryl,
lysyl(N-epsilon-acetyl),
methionyl(sulfone),
methionyl(sulfoxide),
3-(4-methylphenyl)alanyl,
3-(naphth-1-yl)alanyl,
3-(naphth-2-yl)alanyl,
ornithyl,
phenylglycyl,
3-(3-pyridyl)alanyl,
seryl(O-benzyl),
styrylalanyl,
1,2,3,4-tetrahydroisoquinoline-3-carbonyl,
3-(thiazolyl)alanyl,
3-(thien-2-yl)alanyl, and
D-3-(thien-2-yl)alanyl;
Xaa₅ is selected from the group consisting of
isoleucyl,
D-isoleucyl, and
D-leucyl;
Xaa₆ is selected from the group consisting of
seryl, and
threonyl;
Xaa₇ is selected from the group consisting of
glutaminyl,
norvalyl, and
seryl;
Xaa₈ is isoleucyl;
Xaa₉ is arginyl;
Xaa₁₀ is prolyl; and
Xaa₁₁ is selected from the group consisting of
D-alanylamide, and
NH-ethyl.

2. A pharmaceutical composition comprising a compound of claim 1 and a pharmaceutically acceptable carrier.

3. Use of a compound of claim 1 for manufacturing a medicament for treating a patient in need of anti-angiogenesis therapy by administration to the patient in need of a therapeutically effective amount.

4. A composition for the treatment of a disease selected from cancer, arthritis, psoriasis, angiogenesis of the eye associated with infection or surgical intervention, macular degeneration and diabetic retinopathy comprising a compound of claim 1 in combination with a pharmaceutically acceptable carrier.

5. A method of isolating a receptor from an endothelial cell comprising binding a compound of claim 1 to the receptor to form a peptide receptor complex; isolating the peptide receptor complex; and purifying the receptor.

6. A compound according to claim 1 or a pharmaceutically acceptable salt thereof, selected from the group consisting of and

7. A compound according to claim 1, or a therapeutically acceptable salt thereof, selected from the group consisting of 4 and

## Patentansprüche

1. Eine Verbindung mit der Formel (I) oder ein pharmazeutisch verträgliches Salz davon, worin
Xₐₐ₁ gewählt ist aus der Gruppe bestehend aus
Acetyl, und
6-Methylnikotinyl;
Xₐₐ₂ Sarcosyl ist;
Xₐₐ₃ Glycyl ist;
Xₐₐ₄ gewählt ist aus der Gruppe bestehend aus
Alloisoleucyl,
Allylglycyl,
2-Aminobutyryl,
(1R,4S)-1-Aminocyclopent-2-en-4-carbonyl,
3-(5-Bromthien-2-yl)alanyl,
3-(3-Chlorphenyl)alanyl,
3-(4-Chlorphenyl)alanyl,
3-(3-Cyanophenyl)alanyl,
Cysteinyl(S-ethyl),
Cysteinyl(S-methyl),
2,3-Diaminopropionyl,
2,4-Diaminobutanoyl,
3-(3,4-Dimethoxyphenyl)alanyl,
3-(3-Fluorphenyl)alanyl,
3-(4-Fluorphenyl)alanyl,
Homophenylalanyl,
Homoseryl,
Lysyl (N-epsilon-acetyl),
Methionyl(sulfon),
Methionyl(sulfoxid),
3-(4-Methylphenyl)alanyl,
3-(Naphth-1-yl)alanyl,
3-(Naphth-2-yl)alanyl,
Ornithyl,
Phenylglycyl,
3-(3-Pyridyl)alanyl,
Seryl(O-benzyl),
Styrylalanyl,
1,2,3,4-Tetrahydroisochinolin-3-carbonyl,
3-(Thiazolyl)alanyl,
3-(Thien-2-yl)alanyl, und
D-3-(Thien-2-yl)alanyl;
Xₐₐ₅ ist gewählt aus der Gruppe bestehend aus Isoleucyl,
D-Isoleucyl, und
D-Leucyl;
Xₐₐ₆ ist gewählt aus der Gruppe bestehend aus Seryl, und
Threonyl;
Xₐₐ₇ ist gewählt aus der Gruppe bestehend aus Glutaminyl,
Norvalyl, und
Seryl;
Xₐₐ₈ ist Isoleucyl;
Xₐₐ₉ ist Arginyl;
Xₐₐ₁₀ ist Prolyl; und
Xₐₐ₁₁ ist gewählt aus der Gruppe bestehend aus
D-Alanylamid, und
NH-Ethyl.

2. Eine pharmazeutische Zusammensetzung, die eine Verbindung von Anspruch 1 und einen pharmazeutisch verträglichen Träger umfaßt.

3. Verwendung von einer Verbindung von Anspruch 1, zur Herstellung eines Medikaments zur Behandlung eines Patienten, der eine anti-Angiogenesetherapie benötigt, durch Verabreichen an den Patienten, der eine therapeutisch wirksame Menge benötigt.

4. Eine Zusammensetzung für die Behandlung einer Erkrankung gewählt aus Krebs, Arthritis, Psoriasis, Angiogenese des Auges assoziiert mit Infektion oder chirurgischem Eingriff, Makuladegeneration und diabetischer Retinopathie, welche eine Verbindung von Anspruch 1 in Kombination mit einem pharmazeutisch verträglichen Träger umfaßt.

5. Ein Verfahren zum Isolieren eines Rezeptors aus einer Endothelialzelle, welche eine Bindung einer Verbindung von Anspruch 1 zu dem Rezeptor umfaßt, um einen Peptidrezeptorkomplex zu bilden, Isolieren des Peptidrezeptorkomplexes; und Reinigen des Rezeptors.

6. Eine Verbindung gemäß Anspruch 1 oder ein pharmazeutisch verträgliches Salz davon, gewählt aus der Gruppe bestehend aus und

7. Eine Verbindung gemäß Anspruch 1 oder ein therapeutisch verträgliches Salz davon, gewählt aus der Gruppe bestehend aus und

## Revendications

1. Composé de formule (I)
ou sel pharmaceutiquement acceptable de celui-ci, où
Xaa₁ est choisi dans le groupe consistant en
acétyle, et
6-méthylnicotinyle ;
Xaa₂ est sarcosyle ;
Xaa₃ est glycyle ;
Xaa₄ est choisi dans le groupe consistant en
alloisoleucyle,
allylglycyle,
2-aminobutyryle,
(1R,4S)-1-amino-cyclopent-2-ène-4-carbonyle,
3-(5-bromothien-2-yl)alanyle,
3-(3-chlorophényl)alanyle,
3-(4-chlorophényl)alanyle,
3-(3-cyanophényl)alanyle,
cystéinyl(S-éthyle),
cystéinyl(S-méthyle),
2,3-diaminopropionyle,
2,4-diaminobutanoyle,
3-(3,4-diméthoxyphényl)alanyle,
3-(3-fluorophényl)alanyle,
3-(4-fluorophényl)alanyle,
homophénylalanyle,
homoséryle,
lysyl(N-épsilon-acétyle),
méthionyl(sulfone),
méthionyl(sulfoxyde),
3-(4-méthylphényl)alanyle,
3-(napht-1-yl)alanyle,
3-(napht-2-yl)alanyle,
ornithyle,
phénylglycyle,
3-(3-pyridyl)alanyle,
seryl(O-benzyle),
styrylalanyle,
1,2,3,4-tétrahydroisoquinoléine-3-carbonyle,
3-(thiazolyl)alanyle,
3-(thién-2-yl)alanyle, et
D-3-(thién-2-yl)alanyle ;
Xaa₅ est choisi dans le groupe consistant en
isoleucyle,
D-isoleucyle, et
D-leucyle ;
Xaa₆ est choisi dans le groupe consistant en
séryle, et
thréonyle ;
Xaa₇ est choisi dans le groupe consistant en
glutaminyle,
norvalyle, et
séryle ;
Xaa₈ est isoleucyle ;
Xaa₉ est arginyle ;
Xaa₁₀ est prolyle ; et
Xaa₁₁ est choisi dans le groupe consistant en
D-alanylamide, et
NH-éthyle.

2. Composition pharmaceutique comprenant un composé selon la revendication 1 et un support pharmaceutiquement acceptable.

3. Utilisation d'un composé selon la revendication 1 pour fabriquer un médicament pour traiter un patient nécessitant une thérapie anti-angiogenèse par administration au patient nécessitant d'une quantité thérapeutiquement efficace.

4. composition pour le traitement d'une maladie choisie parmi le cancer, l'arthrite, le psoriasis, l'angiogenèse de l'oeil associée avec une infection ou une intervention chirurgicale, la dégénérescence maculaire et la rétinopathie diabétique comprenant un composé selon la revendication 1 en combinaison avec un support pharmaceutiquement acceptable.

5. Procédé d'isolement d'un récepteur à partir d'une cellule endothéliale comprenant la liaison d'un composé selon la revendication 1 au récepteur pour former un complexe peptide-récepteur ; l'isolement du complexe peptide-récepteur ; et la purification du récepteur.

6. Composé selon la revendication 1 ou sel pharmaceutiquement acceptable de celui-ci choisi dans le groupe consistant en et

7. Composé selon la revendication 1 ou sel thérapeutiquement acceptable de celui-ci choisi dans le groupe consistant en et
